# EUROPÄISCHE PATENTSCHRIFT

(11) **EP 0 621 897 B1**
(45) Veröffentlichungstag und Bekanntmachung des Hinweises auf die Patenterteilung: **14.05.1997**
(21) Anmeldenummer: 92921402.1
(22) Anmeldetag: 19.10.1992
(51) Int. Cl.: C12N 9/08

(54) **VEFAHREN ZUR HERSTELLUNG LIGNOLYTISCHER ENZYME MITTELS WEISSFÄULEPILZEN**
PROCESS FOR PREPARING LIGNOLYTIC ENZYMES BY MEANS OF WHITE ROT FUNGI
PROCEDE DE FABRICATION D'ENZYMES LIGNOLYTIQUES AU MOYEN DE CHAMPIGNONS DE POURRITURE BLANCHE

(30) Priorität: 21.10.1991 DE 4134716
(43) Veröffentlichungstag der Anmeldung: 02.11.1994
(73) Patentinhaber: LIGNOZYM GESELLSCHAFT ZUR HERSTELLUNG UND ZUM VERTRIEB VON ENZYMEN mbH, D-52499 Baesweiler (DE)
(72) Erfinder: CALL, Hans-Peter, D-52531 Übach-Palenberg (DE)
(74) Vertreter: Fitzner, Uwe, Dr.
(86) Internationale Anmeldenummer: EP9202398
(87) Internationale Veröffentlichungsnummer: WO9308272

(56) Entgegenhaltungen:
- WO-A-90/04021
- FR-A- 2 600 077
- JOURNAL OF BIOTECHNOLOGY, Bd. 8, Nr. 2, Juni 1988, AMSTERDAM, NL, Seiten 163-170; S. LINKO: 'Continuous production of lignin peroxidase by immobilized Phanerochaete chrysosporium in a pilot scale bioreactor'
- CHEMICAL ABSTRACTS, vol. 109, no. 13, 26. September 1988, Columbus, Ohio, US, abstract no. 108861; M. ASTHER ET AL, Seite 484
- JOURNAL OF BIOTECHNOLOGY, Bd. 8, Nr. 2, Juni 1988, AMSTERDAM, NL, Seiten 97-112; H. JANSHEKAR AND A. FIECHTER: 'Cultivation of Phanerochaete chrysosporium and production of lignin peroxidases in submerged stirred tank reactors'

## Beschreibung

Die vorliegende Erfindung betrifft ein Verfahren zur Herstellung lignolytischer Enzyme mittels Weißfäulepilzen aus der Unterklasse der Aphyllophorales.

In den letzten zehn Jahren ist vielfach versucht worden. Weißfäulepilze im industriellen Maßstab zur Erzeugung lignolytischer Enzyme einzusetzen. Im Mittelpunkt des Interesses stand hierbei Phanerochaete chrysosporium.

So gibt es Versuche im Rührfermenter ( H.Janshekar und A.Fiechter. Journal of Biotechnology. 8 (1988). 97-112 ). Diese Versuche waren jedoch wenig erfolgreich. weil im Rührfermenter die von Phanerochaete chrysosporium gebildeten Pellets immer wieder zerschlagen werden. Da aber Phanerochaete chrysosporium nur dann Enzyme produziert. wenn er in Pelletform oder als Immobilisat vorliegt. läßt sich im Rührfermenter mit den gängigen Verfahren eine optimale Enzymproduktion nicht erreichen.

Darüberhinaus sind in der Bioverfahrenstechnik Reaktoren speziell für empfindliche Zellen entwickelt worden. So ist ein sogenannter Vibromischer entwickelt worden. welcher sich sogar für empfindliche Tier- und Pflanzenzellen eignet ( Einsele. Finn. Samhaber: Mikrobiologische und biochemische Verfahrenstechnik. Weinheim 1985. S.150: Einsele: Chem.Ing.Tech. 45 (1973), 1368: Rehm. Chem. Ing. Tech. 42 (1970). 583). Die Wirkung des Vibromixers beruht auf dem Bernoulli-Effekt. Anstelle eines Rührers befindet sich im Innern des Gefäßes eine waagerecht liegende. an einem senkrechten Schaft befindliche Platte. die sich nach unten verengende Bohrungen enthält. Sie wird mit Hilfe des Schafts auf und nieder bewegt. Dabei entstehen Druckdifferenzen in den Bohrungen. weil die Flüssigkeit in den Öffnungen vom großen zum kleinen Durchmesser zurückfließt. Die Zellen werden auf diese Weise zwar gut bewegt, doch bleiben Schädigungen weitgehend aus. Ein derartiger Vibromixer hat jedoch den Nachteil, daß er für die empfindlichen Pellets von Phanerochaete chrysosporium immer noch nicht schonend genug arbeitet. Außerdem können derartige Vibromixer bis heute noch nicht im großtechnischen Maßstab eingesetzt werden.

Für die Bildung von Pellets von Mikroorganismen sind schließlich auch Glasgefäße geeignet, welche von Schüttelmaschinen bewegt werden ( H.J. Rehm: Einführung in die industrielle Mikrobiologie, Berlin-Heidelberg-New York 19971). Zu diesem Zweck sind die Behältnisse ( zumeist Flaschen oder Erlenmeyerkolben ) in mehreren Etagen übereinander in die Maschinen eingespannt. Durch die Schüttelbewegung der Gefäße wird Luft von der Oberfläche her in die Lösung eingeschlagen. Zum Schütteln verwendet man Amplituden-Ratations- und Vibrationsmaschinen, die normalerweise variierbare Bewegungsgeschwindigkeiten haben. Die Gefäße können mit flächigen Ausbuchtungen, den sogenannten Schikanen, versehen sein. Nachteilig bei diesem Systemen ist, daß sie sich nur für den Labormaßstab eignen. Für die großindustrielle Anwendung wären tausende Flaschen bzw. Erlenmeyerkolben notwendig, so daß die Anwendung derartiger Geräte wirtschaftlich nicht sinnvoll ist.

Um die oben genannten Probleme des Einsatzes von Rührreaktoren zu lösen. hat man verstärkt versucht, mit immobilisierten Zellen zu arbeiten ( S.Linko. Journal of Biotechnology, 8 (1988), 163-170; H.Willerhausen, A. Jäger, H. Graf, Journal of Biotechnology, 6 (1987). 239-243: Y.Linko, M.Leisola, N.Lindholm, J.Troller, P.Linko, A.Fiechter, Journal of Biotechnology 4 (1986), 283-291). Aber auch diese Versuche scheiterten bei größeren Volumina. In der Regel waren diese Verfahren bei mehr als 40 l Fermentervolumen nicht mehr durchführbar. Der Grund ist im wesentlichen darin zu sehen, daß Phanerochaete chrysosporium in Pelletform eine optimale Oberfläche aufweist und daher gerade in diesem Zustand am besten das Enzym produziert.

Um die genannten Probleme bei der Verwendung von Phanerochaete chrysosporium für die Produktion von lignolytischen Enzymen zu lösen. ist ein neuartiger Reaktor speziell entwickelt worden. Dieser ist Gegenstand der vorangemeldeten, aber nicht vorveröffentlichten deutschen Patentanmeldung P 40 12 743. Hierbei handelt es sich um ein rührerloses, geschlossenes Fermentationsgefäß, welches in einer kardanischen Aufhängevorrichtung frei drehbar aufgehängt ist. Unter dem Gefäß ist ein Antriebsmotor angeordnet, dessen Antriebswelle mit dem Boden des Gefäßes mit einem Exzenter verbunden ist. Durch den Exzenter lassen sich Neigungswinkel und Schwingungsamplitude des Gefäßes einstellen. Mittels des stufenlos regelbaren Motors können somit Dreh- und Schwenkbewegungen des Gefäßes erreicht werden.

In dem auf diese Art bewegten Fermenter bildet Phanerochaete chrysosporium Pellets. ohne daß diese durch mechanische Einflüsse fortwährend wieder zerstört werden. Dies hat zur Folge, daß die Pellets eine für die Enzymproduktion optimale Form erreicht. Da andererseits die schwingungsarme kardanische Aufhängung des Reaktors auch den Bau von Reaktorvolumina zwischen ein und drei Kubikmeter gestattet, läßt sich mit der beschriebenen Vorrichtung eine Produktion lignolytischer Enzyme im industriellen Maßstab mit bis dahin nicht für möglich gehaltenen Ausbeuten erreichen.

Die Ergebnisse der Produktion mit diesem Fermenter sind jedoch immer noch nicht zufriedenstellend. Denn die Ausbeuten haben hierbei immer noch nicht ein solches Volumen erreicht, daß sie für eine großindustrielle Produktion lohnend wären.

Die vorliegende Erfindung hat sich daher die Aufgabe gestellt, ein verbessertes Verfahren zur Herstellung lignolytischer Enzyme mittels Weißfäulepilzen, wobei eine aus Pilzen der Unterklasse der Aphyllophorales bestehende Kultur in ein mit Nährlösung gefülltes, geschlossenes Fermentationsgefäß gegeben wird, dort zur Erzeugung von Pellets der Aphyllophorales die Suspension so schonend bewegt wird, daß keine die Pellets zerstörenden Scherkräfte entstehen können, zur Verfügung zu stellen, das auch in Fermentationsgefäßen von mehr als 40 l durchführbar ist und gegenüber dem bisherigen Stand der Technik signifikant gesteigerte Enzymausbeuten gewährleistet.

Diese Aufgabe wird dadurch gelöst, daß unter Zusatz von Hefen oder Benzaldehyden, Chlorbenzaldehyden, Nitrobenzaldehyden, Hydroxybenzaldehyden, Aminobenzaldehyden, Methylbenzaldehyden, Diarylen oder Triarylen, Dialkylalkanen, Trialkyl alkanen, offenkettigen oder cyclischen Iminen oder Derivaten der genannten Stoffe als induktiven Verbindungen, wobei die Induktion einfach oder mehr fach erfolgen kann, die Enzyme erzeugt und danach geerntet werden.

Durch den Einsatz einer aus Aphyllophorales und Hefen bestehenden Mischkultur wird eine erhebliche Steigerung der Enzymproduktion im Vergleich zum bisherigen Stand der Technik erreicht. Die der Kultur zugesetzten Hefen werden hierbei im Laufe des Verfahrens von den Aphyllophorales fast vollständig lysiert. Die erheblichen Mengen an freiwerdenden Hefebestandteilen. z.B. Zellwandbestandteile und Hefeglukane, haben wahrscheinlich Schutz-kolloidcharakter und bewirken so eine Steigerung der Enzymproduktion.

Es ist aus dem Stand der Technik bekannt, daß verschiedene chemische Sustanzen als Induktoren in Fermentationslösungen wirken können. So lassen sich die Laccaseausbeuten z.B. durch Zusatz von Xylidin verbessern. Diese und ähfliche Substanzen sind aber wegen ihrer hohen Toxizität großtechnisch kaum einsetzbar, so daß man von dieser Möglichkeit einer Enzymsteigerung bisher keinen Gebrauch gemacht hat. Erfindungsgemäß wurde nun eine neue Induktorengruppe gefunden, die diese Nachteile nicht aufweist. Es handelt sich hierbei Benzaldehyde, Chlorbenzaldehyde, Nitrobenzaldehyde, Hydroxybenzaldehyde, Aminobenzaldehyde, Methyl-benzaldehyde,Diaryle, Triaryle, Dialkylalkane, Trialkylalkane , offenkettige oder cyclische Imine. Ebenso können die Derivate der genannten Verbindungen verwendet werden. Diese Stoffe weisen überraschenderweise nicht die genannten Nachteile auf. Insbesondere sind sie aber auch auch für den Einsatz im großtechnischen Maßstab geeignet. Überraschend ist insbesondere, daß sich durch den Einsatz der genannten Induktoren sehr hohe Enzymausbeuten erreichen lassen (ca. 2000 IU/ml Enzym).

Die geschilderten überraschend verbesserten Enzymausbeuten können bereits bei Einsatz der Gemische aus Aphyllophorales mit Hefen oder den aufgezählten Induktoren in herkömmlichen Rührfermentern beobachtet werden. Diese müssen allerdings mit einem speziellen Rührer ausgestattet sein, dessen Paddel sich verstellen lassen, daß beim Rühren kaum scherkräfte entstehen, welche zu einer Zerstörung der gebildeten Pellets führen können. Erfindungsgemäß wichtig ist daher zum einen die Gestalt des Rührers und zum anderen die Möglichkeit des Verstellens der Paddel.

Besonders gut sind jedoch die Enzymausbeuten, wenn der oben beschriebene Reaktor gemäß der Deutschen Patentanmeldung P 40 12743 eingesetzt wird. Denn die bei Einsatz dieses Fermenters bereits signifikant erhöhten Enzymausbeuten lassen sich in überraschender Weise nochmals steigern durch Einsatz eines Gemisches aus Apyllophorales und Hefen oder den speziellen induktiv wirkenden Ver bindungen, wobei die Induktion einfach oder mehrfach erfolgen kann.

In einer Variante des Verfahrens kann hierbei so vorgegangen werden, daß zunächst in dem Schüttelfermenter die Pellets unter Zugabe von Hefe oder Benzaldehyden erzeugt werden und im Anschluß hieran die Pellets enthaltende Suspension in einen Rührfermenter überführt wird. Hier werden dann die Enzyme mittels der Pellets produziert. Als vorteilhaft ist weiterhin anzusehen, daß nach Abschluß der En-zymproduktion im Schüttelfermenter oder im Rührfermenter sich die abgetrennten Pellets der Aphyllophorales unmittelbar wieder für die Enzymproduktion einsetzen lassen. Zweckmäßigerweise trennt man hierfür die Pellets von der restlichen Suspension ab und führt sie nach Abtrennung des Enzyms in den Fermenter zurück. Die Abtrennung kann erfindungsgemäß mittels Ultrafiltration durchgeführt werden. In einer erfindungsgemäß bevorzugten Ausführungsform wird Phanerochaete chrysosporium im Gemisch mit Hefen eingesetzt. Hierbei können beim Einsatz im Rührfermenter Spitzenwerte von ca. 1000 IU/pro l Kultur erreicht werden. Beim Einsatz im Schüttelfermenter liegen diese Werte bei 1500 - 2000 IU/l (1IU = Umsatz pro 1 micromol veratrylaalkohol/min./ml Enzym in Veratrylaldehyd).

Vor Durchführung des erfinäungsgemäßen Verfahrens kann Phanerochaete chrysosporium zweckmäßigerweise in Petrischalen und dann in Standkulturen angezüchtet werden. Die gebildeten Mycele werden im Anschluß hieran zerschlagen und in dem erfindungsgemäßen Verfahren eingesetzt. Die Anzucht von Phanerochaete chrysosporium erfolgt unter bekannten Bedingungen. D.h. es wird üblicherweise bei Temperaturen von 37 - 40 ° C und einem pH Wert von 4.5 bis 5 gearbeitet. Der bevorzugte pH Wert liegt bei 5. Als Anzuchttemperatur werden 37 ° C bevorzugt.

Als Substrat für die Kultur von Phanerochaete chrysosporium im Gemisch mit Hefen hat sich die folgende Zusammensetzung bewährt:

Vorkulturmedium:
1) Mineralsalzlösung (ME): (pro Liter)
   10.5 g Nitrotriacetat: 21 g MgSO₄ ; 3.5 g MnSO₄; 7 g NaCl; 0,7 g FeSO₄; 0.7 g CoCl₂; 0.7 g CaCl₂; 0.7 g ZnSO₄; 0.07 g CuSO₄; 0.07 g CuSO₄; 0.07 g AlK₂SO₄; 0.07 g H₃BO₃; 0.07 g NaMoO₄.
2) Lösung 1 (Salzlösung) (pro Liter)
   20 g KH₂PO₄; 5 g MgSO₄; 1 g CaCl₂.
3) Puffer (pH 4,5-5,5)
   1 m NaH₂PO₄ / Na₂HPO₄ - Puffer.

| Zusammensetzung pro 1 l Medium: | |
|---|---|
| 0,2 g | Ammoniumtartrat |
| 100 ml | Lösung 1 |
| 1,43 ml | ME-Lösung |
| 10 g | Glukose |
| 10 ml | Puffer |
| +0,9 ml | Thiamin 100 mg/l (wird nach dem Autoklavieren sterilfiltriert zugesetzt). |

### Hauptkulturmedium:

| Zusammensetzung pro 1 Medium: | |
|---|---|
| 0,2 g | Ammoniumtartrat |
| 100 ml | Lösung 1 |
| 10 ml | ME-Lösung |
| 10 g | Glukose |
| 10 ml | Puffer |
| 67,3 mg | Veratrylalkohol |
| 2 ml | Tween 80 |
| +0,9 ml | Thiamin (100 mg/l). |

In einer weiteren bevorzugten Ausführungsform der Erfindung wird Coriolus versicolor in Kombination mit den oben aufgezählten Induktoren insbesondere Benzaldehyden oder deren Derivaten eingesetzt. Es ist aus dem Stand der Technik bekannt, daß sich gute Laccaseausbeuten mit Induktoren. z.B. Xylidin erreichen lassen. Doch aus den oben genannten Gründen haben sich diese Induktoren nicht bewährt. Durch Einsatz der erfindungsgemäßen Induktoren in Kombination mit Coriolus versicolor konnten nunmehr in überraschenderweise Steigerungen der Enzymausbeuten bis zu 100% erreicht werden. Im folgenden wird die Erfindung unter Bezugnahme auf die Figuren näher beschrieben:
Figur 1 zeigt den erfindungsgemäß eingesetzten rührerlosen Fermenter.
Figur 2a zeigt die spezielle Ausgestaltung eines Rührers für den Einsatz in dem erfindungsgemüßen Verfahren in der Ansicht von oben.
Figur 2b zeigt den Rührer in der Queransicht.
Figur 2c zeigt den Rührer in der Ansicht von der Seite.

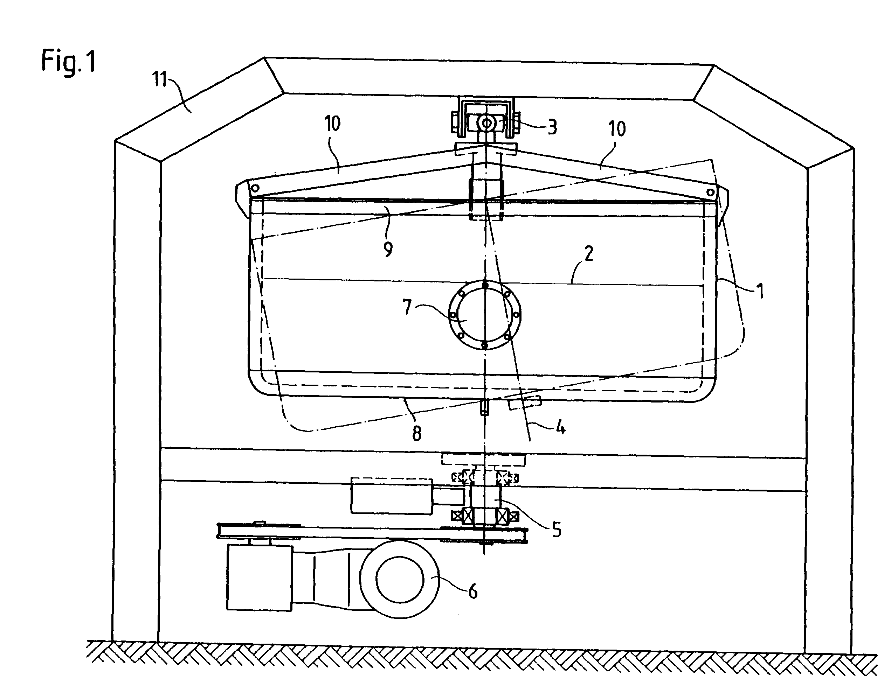

In dem in Figur 1 dargestellten rührerlosen Reaktor erfolgt in erster Linie die Herstellung der Pellets der Aphyllophorales und gegebenenfalls die Herstellung der Enzyme. Hierbei erfolgt die Bildung der Pellets in dem Gefäß 1. Das Gefäß 1 ist ein geschlossener Behälter mit festem Boden 8. Seitenwänden. Deckel 9 und einer Sichtscheibe 7. Seitlich oder am Boden sind Öffnungen für diverse Meßfühler (O₂, PH. Antischaum) sowie für Zu- und Ablauf von Substrat und Impfkultur vorgesehen. Es ist in einem Gestell 11 aufgehängt. Die Befestigung erfolgt hierbei mittels der Greifarme 10. Über diese Greifarme 10 ist das Gefäß 1 an der kardanischen Aufhängevorrichtung frei dreh- und schwenkbar aufgehängt. Die Drehund Schwenkbewegungen werden durch den Motor 6 bewirkt. Dieser ist über die Antriebswelle 5 und den Exzenter 4 mit dem Boden 8 des Gefäßes 1 verbunden. Mittels des Exzenters 4 werden Neigung und Schwingamplitude des Gefäßes 1 eingestellt. Die Geschwindigkeit der Dreh- und Schwenkbewegungen des Gefäßes 1 läßt sich über die Motordrehzahl regeln.

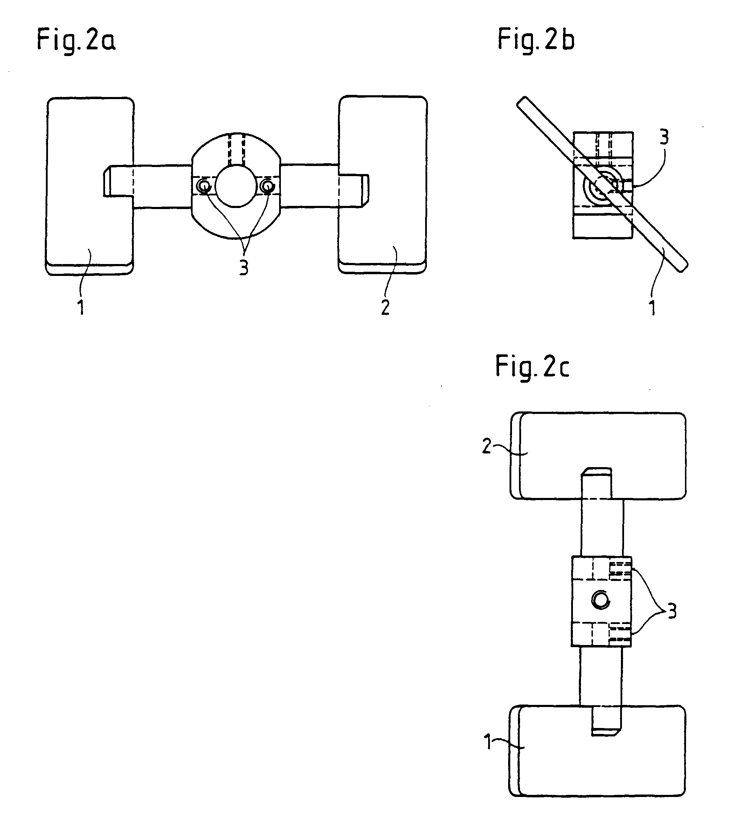

Der in den Figuren 2a-2c dargestellte Rührer ist den beiden Paddeln 1 und 2 ausgestattet. Deren Neigungswinkel läßt sich mit Hilfe der Verstellschraube 3 verändern. Auf diese weise läßt sich der Rührer jeweils exakt entsprechend der Empfindlichkeit der eingesetzten Zellen einstellen, so daß keine die Pellets zerstörenden Scherkräfte auftreten können.

Die Erfindung wird weiterhin anhand folgender Beispiele erläutert:

### Beispiel 1:

Als Stamm wird Phanerochaete chrysosporium ATCC 32629 genommen. Zunächst werden Malzagarplatten beimpft und ca. 10-12 Tage bei 27° C bebrütet. Von diesen Platten wird die Hälfte des Rasens abgenommen und eine 50 ml Standkultur in 500 ml Erlenmeyerkolben beimpft (Bebrütungszeit ca. 5 Tage bei 37° C). Die so erhaltene Vorkultur wird abdekantiert, wieder auf das ursprüngliche Volumen mit a dest, aufgefüllt und daraufhin in einem Braun Starmix für 2 x 30 Sec. auf Stufe 3 zerkleinert. Pro Liter Medium im Schüttelreaktor werden 15 ml Vorkultur zugegeben. d.h.. auf einen 10 l Schüttelreaktor mit 5 l Befüllung 75 ml. An Hefen werden 0.5-0.8 x 2-4 x 10⁵ Hefen/ml Medium zugegeben. Die Anzuchttemperatur beträgt 38°C, die Schüttelfrequenz 90 rpm und die Auslenkung ca. 5cm. Es wird jeden Tag für 30 Sec. mit O₂ (100 l pro Std.) begast. Die Anzuchtdauer beträgt 4-5 Tage. Die Enzymausbeuten betragen ca. 1500-2000]U/ 1 (1TU = 1 µ Mo1 Umsatz von Veratryalkohol in Veratryldehyd/Min.).

Die Medien sind wie folgt zusammengesetzt:

Vorkulturmedium:
1) Mineralsalzlösung (ME): (pro Liter)
   10,5 g Nitrotriacetat; 21 g MgSO₄; 3,5 g MnSO₄; 7 g NaCl. 0,7 g FeSO₄; 0,7 g COCl₂; 0,7 g CaCl₂; 0,7 g ZnSO₄; 0,07 g CuSO₄; 0,07 g CuSO₄; 0,07 g AlK₂SO₄; 0,07 g H₃BO₃; 0,07 g NaMoO₄
2) Lösung 1 (Salzlösung) (pro Liter)
   20 g KH₂PO₄; 5 g MgSO₄; 1 g CaCl₂.
3) Puffer (pH 4.5-5.5)
   1 m NaH₂PO₄/ Na₂HPO₄ - Puffer.

| Zusammensetzung pro 1 l Medium: | |
|---|---|
| 0,2 g | Ammoniumtartrat |
| 100 ml | Lösung 1 |
| 1,43 ml | ME-Lösung |
| 10 g | Glukose |
| 10 ml | Puffer |
| +0,9 ml | Thiamin 100 mg/l (wird nach dem Autoklavieren sterilfiltriert zugesetzt). |

### Hauptkulturmedium:

| Zusammensetzung pro 1 Medium: | |
|---|---|
| 0,2 g | Ammoniumtartrat |
| 100 ml | Lösung 1 |
| 10 ml | ME-Lösung |
| 10 g | Glukose |
| 10 ml | Puffer |
| 67,3 mg | Veratrylalkohol |
| 2 ml | Tween 80 |
| +0,9 ml | Thiamin (100 mg/l). |

### Beispiel 2:

Als Stamm wird Phanerochaete chrysosporium ATCC 32629 genommen. Zunächst werden Malzagarplatten beimpft und ca. 10-12 Tage bei 27° C bebrütet. Von diesen Platten wird die Hälfte des Rasens abgenommen und eine 50 ml Standkultur in 500 ml Erlenmeyerkolben beimpft (Bebrütungszeit ca. 5 Tage bei 37° C). Die so erhaltene Vorkultur wird abdekantiert, wieder auf das ursprüngliche Volumen mit a.dest. aufgefüllt und daraufhin in einem Braun Starmix für 2 x 30 Sec. auf Stufe 3 zerkleinert. Pro Liter Medium im Rührreaktor werden 15 ml Vorkultur zugegeben. d.h.. auf einen 10 l Schüttelreaktor mit 5 l Befüllung 75 ml. Als Hefe werden 2-4 x 10⁵ Hefen/ml Medium zugegeben. Die Anzuchttemperatur beträgt 38 ° C. die Rührerdrehzahl 110 rpm.

Der Sauerstoff Partialdruck der Kultur wird mit Hilfe einer O₂-Messung mittels 02-Elektrode und Steuerung auf 30-70 % gehalten. Die Anzuchtdauer beträgt 4-5 Tage. Die Enzymausbeuten betragen bis 1000 IU/l.

### Beispiel 3:

Als Stamm wird der Stamm Coriolus versicolor ATCC 34671 verwendet. Von Malzschrägagarröhrchen wird mit Hilfe eines Korkbohrers ein ca. 1 cm φ großes Mycelagarstück auf die Mitte einer Malzagarplatte gebracht und ca. 8 Tage bebrütet (28° C). Von dieser Platte werden je drei 1 cm φ Stücke ausgestanzt und mit der Mycelseite nach oben auf eine 50 ml Standkultur (im 300 ml Erlenmeyerkolben) gebracht. Bebrütung ca. 8 Tage bei 28° C. Der gebildete Mycelrasen wird von der Standkulturflüssigkeit getrennt und für 2 x 30 Sec. in einem Braun Starmix gemixt (Stufe 3).

Pro l Medium werden 30-60 ml Vorkulturmedium als Inokulum verwendet. Als Induktor wird nach 3-4 Tagen Anzuchtzeit 10-20 ml einer 1 %-igen Lösung von 2-Aminobenzaldehyd in 50 %-igen Alkohol verwendet. Die gesamte Anzuchtzeit beträgt 6 Tage.

Als Medium kommt die im folgenden wiedergegebene Zusammensetzung in Betracht:

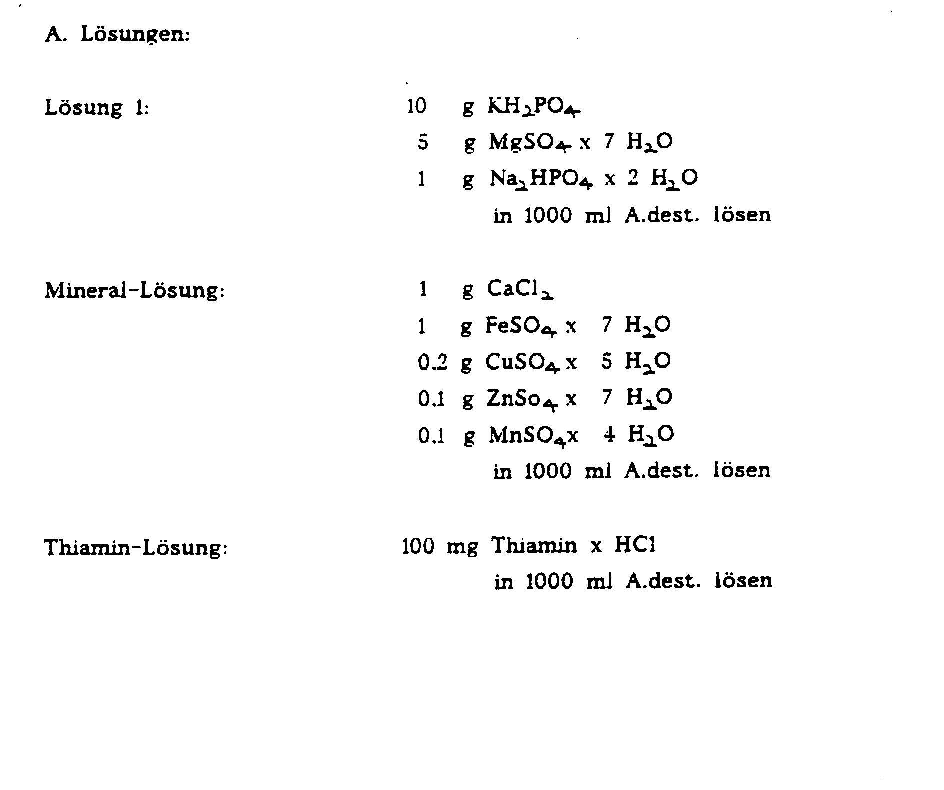

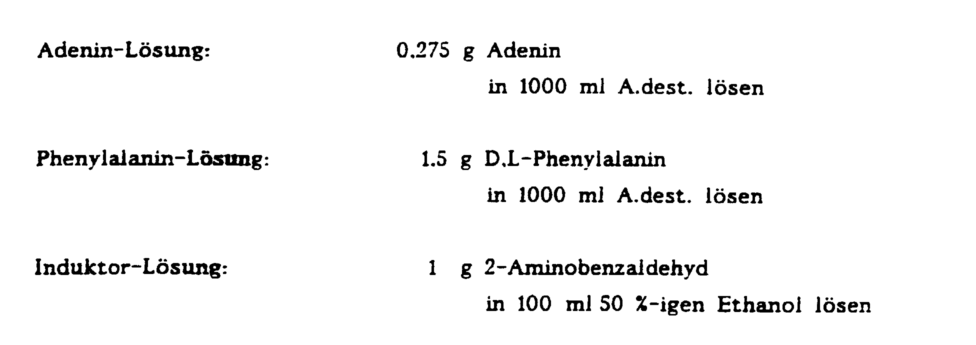

| B. Medien: | | |
|---|---|---|
| Vorkultur-Medium: | 20 g | Glucose |
| | 2,5 g | L-Aspargagin |
| | 100 ml | Lösung |
| | 10 ml | Mineral-Lösung |
| | 100 ml | Adenin-Lösung |
| | 100 ml | Phenylalanin-Lösung mit A.dest. auf 1000 ml auffüllen. den pH-Wert mit 1 M HC1 auf pH 5.0 einstellen. |
| | | |
| | 0.5 ml | Thiamin-Lösung nach dem Autoklavieren sterilfiltriert zugeben. |
| | | |
| Hauptkultur-Medium: | | entspricht dem Vorkultur-Medium mit folgendem Zusatz auf 1000 ml: |
| | | |
| | | 10-20 ml einer 1 %-igen Lösung von z.B. 2-Aminobenzaldehyd in 50 %-igen Athanol. |

## Patentansprüche

1. Verfahren zur Herstellung lignolytischer Enzyme mittels Weißfäulepilzen, wobei eine aus Pilzen der Unterklasse der Aphyllophorales bestehende Kultur in ein mit Nährlösung gefülltes, geschlossenes Fennentationsgefäß gegeben wird, dort zur Erzeugung von Pellets der Aphyllophorales die Suspension so schonend bewegt wird, daß keine die Pellets zerstörenden Scherkräfte entstehen können,
**dadurch gekennzeichnet, daß**
unter Zusatz von Hefen oder Benzaldehyden, Chlorbenzaldehyden, Nitrobenzaldehyden, Hydroxybenzaldehyden, Aminobenzaldehyden, Methylbenzaldehyden, Diarylen, Triarylen, Dialkylalkanen, Trialkylalkanen, offenkettigen oder cyclischen Iminen oder Derivaten der genannten Stoffe als induktiven Verbindungen, wobei die Induktion einfach oder mehrfach erfolgen kann, die Enzyme erzeugt und danach geerntet werden.

2. Verfahren nach Anspruch 1,
**dadurch gekennzeichnet, daß**
nach Abschluß der Enzymproduktion die Pellets von dem flüssigen Nährmedium abgetrennt und erneut zur Enzymproduktion in den Fermenter zurückgeführt werden.

3. Verfahren nach einem der Ansprüche 1 oder 2,
**dadurch gekennzeichnet, daß**
die Kultur in einen Fermenter gegeben wird, welcher mit einem Rührer ausgestattet ist, dessen Paddel so einstellbar sind, daß keine die Pellets zerstörenden Scherkräfte entstehen.

4. Verfahren nach einem der Ansprüche 1 bis 3,
**dadurch gekennzeichnet, daß**
Phanerochaete chrysosporium unter Zusatz von Hefen in das Fennentationsgefäß gegeben wird.

5. Verfahren nach einem der Ansprüche 1 bis 4,
**dadurch gekennzeichnet, daß**
Coriolus versicolor unter Zusatz von Benzaldehyden, Chlorbenzaldehyden, Nitrobenzaldehyden, Hydroxybenzaldehyden, Aminobenzaldehyden, Methylbenzaldehyden , Diarylen, Triarylen , Dialkylalkanen, Trialkyl alkanen, offenkettigen oder cyclischen Iminen oder Derivaten der genannten Stoffe als induktiven Verbindungen, wobei die Induktion einfach oder mehrfach erfolgen kann, die Enzyme erzeugt.

## Claims

1. Process for producing lignolytic enzymes by means of white rot fungi, a culture consisting of fungi of subclass Aphyllophorales being placed into a closed fermentation vessel charged with nutrient solution, and the suspension there, to produce pellets of Aphyllophorales, being agitated so gently that no shear forces destroying the pellets can arise, characterized in that the enzymes are produced with addition of yeasts or benzaldehydes, chlorobenzaldehydes, nitrobenzaldehydes, hydroxybenzaldehydes, aminobenzaldehydes, methylbenzaldehydes, diarylene, triarylene, dialkylalkanes, trialkyl-alkanes, open-chain or cyclic imines or derivatives of the said substances, as inducing compounds, with the induction being able to be performed once or repeatedly, and the enzymes are then harvested.

2. Process according to Claim 1, characterized in that, after completion of the enzyme production, the pellets are separated off from the liquid nutrient medium and recycled to the fermenter again for enzyme production.

3. Process according to one of Claims 1 or 2, characterized in that the culture is placed into a fermenter which is equipped with an agitator whose paddles can be set in such a manner that no shear forces destroying the pellets arise.

4. Process according to one of Claims 1 to 3, characterized in that Phanerochaete chrysosporium is placed into the fermentation vessel with addition of yeasts.

5. Process according to one of Claims 1 to 4, characterized in that Coriolus versicolor, with addition of benzaldehydes, chlorobenzaldehydes, nitrobenzaldehydes, hydroxybenzaldehydes, aminobenzaldehydes, methylbenzaldehydes, diarylene, triarylene, dialkylalkanes, trialkylalkanes, open-chain or cyclic imines or derivatives of the said substances as inducing compounds, with the induction being able to be performed once or repeatedly, produces the enzymes.

## Revendications

1. Procédé pour la production d'enzymes lignolytiques au moyen de moisissures blanches, dans lequel on introduit une culture, constituée de champignons appartenant à la sous-classe des Aphyllophorales, dans un récipient de fermentation clos, contenant une solution nutritive, on y agite de façon ménagée la suspension, pour la production d'agglomérats des Aphyllophorales, de manière qu'il ne puisse en résulter de forces de cisaillement détruisant les agglomérats, caractérisé en ce que l'enzyme est produite avec addition de levures ou de benzaldéhydes, chlorobenzaldéhydes, nitrobenzaldéhydes, hydroxybenzaldéhydes, aminobenzaldéhydes, méthylbenzaldéhydes, diaryles, triaryles, dialkylalcanes, trialkylalcanes, d'imines cycliques ou à chaîne ouverte ou de dérivés des substances citées, en tant que composés inducteurs, l'induction pouvant s'effectuer une ou plusieurs fois, et est ensuite recueillie.

2. Procédé selon la revendication 1, caractérisé en ce qu'une fois terminée la production d'enzyme, les agglomérats sont séparés du milieu nutritif liquide et renvoyés à nouveau dans le fermenteur pour la production d'enzyme.

3. Procédé selon la revendication 1 ou 2, caractérisé en ce que la culture est introduite dans un fermenteur qui est muni d'un agitateur dont les pales sont ajustables de telle façon qu'il n'en résulte pas de forces de cisaillement détruisant les agglomérats.

4. Procédé selon l'une des revendications 1 à 3, caractérisé en ce que l'on introduit dans le récipient de fermentation *Phanerochaete chrysosporium* avec addition de levures.

5. Procédé selon l'une des revendications 1 à 4, caractérisé en ce que *Coriolus versicolor,* avec addition de benzaldéhydes, chlorobenzaldéhydes, nitrobenzaldéhydes, hydroxybenzaldéhydes, aminobenzaldéhydes, méthylbenzaldéhydes, diaryles, triaryles, dialkylalcanes trialkylalcanes , d'imines cycliques ou à chaîne ouverte ou de dérivés des substances citées, en tant que composés inducteurs, l'induction pouvant s'effectuer une ou plusieurs fois, produit les enzymes.
